# EUROPEAN PATENT SPECIFICATION

(11) **EP 2 052 757 B1**
(45) Date of publication and mention of the grant of the patent: **03.07.2013**
(21) Application number: 07742802.7
(22) Date of filing: 02.05.2007
(51) Int. Cl.: A61M 37/00

(54) **MEDICAL CAPSULE**
MEDIZINISCHE KAPSEL
CAPSULE MÉDICALE

(30) Priority: 05.05.2006 DE 102006021016; 11.03.2007 DE 102007011556; 05.04.2007 DE 102007016527
(43) Date of publication of application: 29.04.2009
(73) Proprietor: Olympus Medical Systems Corp., Tokyo 151-0072 (JP)
(72) Inventor: DUERSCHINGER, Guenter, Fuerth 90768 (DE)
(74) Representative: von Hellfeld, Axel
(86) International application number: PCT/JP2007/059367
(87) International publication number: WO 2007/129655

(56) References cited:
- WO-A-2004/005156
- GB-A- 2 356 842
- JP-A- 05 168 680
- JP-A- 07 111 985
- JP-A- 2002 186 672
- JP-A- 2002 186 672
- US-A- 4 793 997
- US-B1- 6 283 953

## Description

### TECHNICAL FIELD

The present invention relates to a medical capsule being able to be chemically activated for especially testing patency of intestinal tract or treating of the intestinal tract, to ensure that a subsequent examination with a video capsule can be carried out.

### BACKGROUND ART

Video capsules for examining an intestinal tract for bleeding, inflammations, cancer etc. are described in Patent Documents 1 to 7.

For example, Fig. 4 in Patent Document 7 shows a device 50 useful for testing patency of the intestinal tract. This device 50 has a needle-shaped radio frequency identifier (denoted by 53) 2mm in diameter and 12 mm tall coated with a glass, which is not dissolved by the digestive secretions of the intestinal tract. Accordingly, the device 50 needs to pass through a bottleneck existing in the intestinal tract before being excreted naturally.

Patent Document 8 describes a capsule having a needle-shaped casing 2 made of a material resistant to all digestive secretions of human body.

In the course of endoscopical treatment or examination often a colouring of the upper and lower gastrointestinal tract (GI) is performed, for example with indigocarmine, because such colouring improves visibility of the inner intestine wall and the mucosa to determine vessel disease or other structures.

Patent Document 1: United States Patent Publication No. 2001/0035902 Description
Patent Document 2: United States Patent Publication No. 2002/0103417 Description
Patent Document 3: United States Patent Application Publication No. 2002/0165592 Description
Patent Document 4: United States Patent No. 4,278,077 Description
Patent Document 5: United States Patent No. 6,240,312 Description
Patent Document 6: European Patent Application Publication No. 1 604 606 Description
Patent Document 7: International Publication No. 03/005887 Pamphlet
Patent Document 8: Germany Utility-model No. 202004 020433 Description
GB 2 356 842 A discloses a capsule made by injection-moulding from one or more of a range of thermoplastic materials, typically a soluble polyvinylalcohol, and is constructed to dissolve at varying rates in an aqueous environment. The capsule consists of a body and a cap, which form two compartments side by side.
US 4,793,997 discloses a device for the controlled release of an active material into an aqueous medium, e.g. body fluids, comprising a water soluble glass container in which the active material is sealed. The container wall has a region of reduced thickness, which region dissolves before the remainder of the container wall to release the active material.
WO 2004/005156 A1 discloses a water-soluble container comprising one or more discrete chambers for containing a product. At least a part of the wall or at least one of the chambers is adapted to dissolve before the remainder of the chamber to allow the product to escape.

### DISCLOSURE OF INVENTION

### PROBLEM TO BE SOLVED BY THE INVENTION

However, Patent Documents 1 to 7 have a common problem that a video capsule may be not dissolved by the digestive secretions of the intestinal tract and thus stuck in a bottleneck in the intestinal tract. A stuck capsule usually needs to be removed surgically.

Specifically, for example, this needle-shaped body 53 described in Patent Document 7 can jackknife and hook or stuck at a bottleneck of the intestinal tract or the intestinal villi. In that case, an operation can be necessary. The coating 54 of the device 50 is impenetrable to stomach and intestinal liquid; however, at the time of being discharged completely, the coating 54 is squashed to small by the pressure in the intestinal tract and has to pass through an existing bottleneck in that form.

This needle-shaped casing described in Patent Document 8 can jackknife, hook or stuck at a bottleneck of the intestinal tract or the intestinal villi.

Also, with respect to middle part of stomach, conventional endoscopes can only reach its entrance. In addition, examination of the small intestine has been performed approximately 4 to 5 years ago with the video capsule or with a double-balloon endoscope.

The present invention is made in view of the foregoing. An object of the invention is to provide a capsule in which neither a needle-shaped internal body nor a coating made of a material which cannot be dissolved by the digestive secretions exists. In other words, although a capsule dissolves completely at a bottleneck, the capsule makes a patency test possible and at the same time certainly indicates an existing bottleneck in a clear way.

Another object of the invention is to provide a capsule which can perform colouring of an inner wall of the middle part of stomach.

### MEANS FOR SOLVING PROBLEM

To solve the problems and achieve the objects, an aspect according to the present invention is a medical capsule, having a capsule shape and size capable of being introduced into a subject body, at least a part of the medical capsule being dissolved over time by remaining in a predetermined time or more in a lumen. The medical capsule includes inner and outer casing, for forming the capsule shape, having at least two thin portions, wherein a communication opening for communicatively connecting an inner space of the casing to an outside of the medical capsule is formed when the thin portion is eliminated earlier than other portions of the medical capsule by dissolution of the casing.

Another aspect according to the description is a medical capsule, having a capsule shape and size capable of being introduced into a subject body, at least a part of the medical capsule being dissolved over time by remaining in a predetermined time or more in a lumen. The medical capsule includes a casing, for forming the capsule shape, having a thin portion; and a filling with which an inner space of the casing is filled, wherein the filling is discharged to an outside of the medical capsule after dissolution of the thin portion, with an portion of the casing other than the thin portion keeping substantially the capsule shape.

Still another aspect according to the description is a method for testing patency of an intestinal tract with a medical capsule having a capsule shape and size capable of being introduced into a subject body, at least a part of the medical capsule being dissolved over time by remaining in a predetermined time or more in a lumen. The method includes using as the medical capsule a capsule in which at least two thin portions of a casing forming the capsule shape is dissolved after a predetermined time to form a communication opening for communicatively connecting an inner space of the casing to an outside of the medical capsule, and the capsule keeps the capsule shape further after a predetermined time.

Still another aspect according to the description is a method for testing patency of an intestinal tract with a medical capsule having a capsule shape and size capable of being introduced into a subject body, at least a part of the medical capsule being dissolved over time by remaining in a predetermined time or more in a lumen. The method includes using as the medical capsule a capsule, the capsule including a casing, for forming the capsule shape, having a thin portion; and a filling with which an inner space of the casing is filled, wherein the filling is discharged to an outside of the capsule after dissolution of the thin portion, with an portion of the casing other than the thin portion keeping substantially the capsule shape.

Still another aspect according to the description is a method for treating an intestinal tract with a medical capsule having a capsule shape and size capable of being introduced into a subject body, at least a part of the medical capsule being dissolved over time by remaining in a predetermined time or more in a lumen. The method includes using as the medical capsule a capsule, the capsule including a casing, for forming the capsule shape, having a thin portion; and a filling with which an inner space of the casing is filled, wherein the filling is discharged to an outside of the capsule after dissolution of the thin portion, with an portion of the casing other than the thin portion keeping substantially the capsule shape.

Still another aspect according to the description is a medical capsule, having a capsule shape and size capable of being introduced into a subject body, at least a part of the medical capsule being dissolved over time by remaining in a predetermined time or more in a lumen. The medical capsule includes a casing, for forming the capsule shape, having an promptly-dissolving portion, wherein a communication opening for communicatively connecting an inner space of the casing to an outside of the medical capsule is formed when the promptly-dissolving portion is eliminated earlier than other portions of the promptly-dissolving portion by dissolution of the promptly-dissolving portion.

Still another aspect according to the description is a medical capsule for testing patency of an intestinal tract or treating an intestinal tract. The medical capsule includes a first state of dimension such that the capsule passes through an intestinal tract having no narrow area but is stopped by a narrow area; a second state of dimension such that the capsule can pass the narrow area of the intestinal tract; and an indicator material covered by a portion of the capsule hindering the indicator material to flow into the intestine in the first state and flows into the intestine in the second state. The portion of the capsule is dissolvable by secretions of the intestine after a predetermined time to allow the indicator material to flow into the intestine.

Still another aspect according to the description is a medical capsule for coloring an intestinal wall. The medical capsule includes a portion which is non-dissolvable by secretions of a stomach but which is dissolvable by secretions of an intestine after a predetermined time; and a dye covered by the portion in the medical capsule, not flowing out of the capsule when the portion is not dissolved but flowing out of the capsule when the portion is dissolved.

Still another aspect according to the description is a system including a video capsule for imaging an intestinal tract; and a medical capsule according to the description.

### EFFECT OF THE INVENTION

A medical capsule according to the present invention allows a patency test of the intestinal tract and at the same time certainly indicates a narrow-area in a clear way.

A medical capsule according to the present invention allows colouring of an inner wall of the middle part of stomach.

### BRIEF DESCRIPTION OF DRAWINGS

Fig. 1 is a medical capsule with an inner casing having bores at both ends and an outer casing arranged around the inner casing.
Fig. 2 is a medical capsule with a casing formed by connecting an upper portion and lower portion and having thin wall portions at both ends.
Fig. 3 is a medical capsule with an inner casing having bores at both ends and an outer casing formed by connecting with two cup-shaped members and having thin wall portions at both ends.
Fig. 4 is a medical capsule formed by a hollow body and a continuous tube inserted into the hollow body.
Fig. 5 is a medical capsule formed by a hollow body with bores at both ends.
Fig. 6 is a medical capsule formed by a hollow body having a through-bore extending over the entire length.
Fig. 7 is a medical capsule formed by a hollow body having bores at both ends with plugs closing the bores.

### EXPLANATIONS OF LETTERS OR NUMERALS

10, 20, 30, 40, 50, 60, 70 Medical capsule
12, 32 Inner casing
13, 33, 57, 78 Discharge outlets
14, 36, 37 Outer casing
15, 25, 35, 75 Filling
26 Lower portion of casing
27 Upper portion of casing
28, 38 Shaped portion
36, 37 Casing-cup-shaped member
43 Continuous tube
45 Space
56, 72 Hollow body
68 Casing body
69 Bore
73 Plug

### BEST MODE(S) FOR CARRYING OUT THE INVENTION

Exemplary embodiments of a medical capsule according to the present invention will be described in detail below with reference to the drawings. The invention is not limited to the embodiments.

A basic concept of a first embodiment is to provide a medical capsule which is chemically activated. The medical capsule has a capsule shape and size capable of being introduced into the subject body. The medical capsule also has at least an inner casing with two discharge outlets (openings) facing each other and at least a sealed outer casing. The inner and outer casings are made of a material which is dissolved after a predetermined time by digestive secretions of the intestinal tract (lumen). The inner casing is filled with a filling. The filling can flow from the discharge outlets into the intestinal tract following the dissolution of the outer casing no matter which side of the capsule arrives at a bottleneck. Due to the arrangement of the facing discharge outlets, the discharge ability of the filling is preferably influenced by the contraction of the small intestine. The filling may consist of, for example, granulated material, powder, gel or liquid.

An example of the filling is billowing bubble generated when the filling is discharged in the intestinal tract. The bubble passes through the bottleneck or the narrowed area (hereinafter, "narrowed area" includes "bottleneck"), and an intestinal tract without staying there, and is then excreted from the anus. Another example of the filling is gas with an exceptionally striking smell generated when it is discharged in an intestinal tract. This gas immediately passes through the narrowed area without staying there like wind, and flows out from the anus through the intestinal tract. As still another example, the filling may be discharged as a gel or a liquid which dyes the stool with a particularly striking colour. Alternatively, when the intestinal tract is empty, the filling may be naturally excreted in the form of a liquid dyed with a particularly striking colour.

Due to these characteristics, it is certainly acknowledged that the capsule has stuck at a narrow area, and that the outer casing has dissolved completely or partially. The inner casing remains at a narrow-area for a predetermined time until it is completely dissolved. The position of the inner casing can be determined using X-ray technology if a contrast medium such as barium sulphate is homogeneously-mixed and added to the material of the inner casing. A painkiller or other medicine may be added into the filling in the capsule.

As another embodiment, a medical capsule may be formed from only one filled casing made of the same material as the inner casing described above. This casing includes a lower portion and an upper portion which are hermetically joined to each other. The wall thickness of the casing is designed to thin by shaping the interior. As a result, the walls (thin wall portions) dissolve earlier than other portions, and the filling can flow into the intestinal tract. The emptied casing remains stuck at the narrow area for a predetermined time until it is completely dissolved. The location of the casing can be detected with X-ray technique if a contrast medium, for example, is homogeneously-mixed and added to the material of the casing.

If the capsule is naturally excreted from the intestinal tract without being dissolved and with keeping the original shape, the intestinal tract has patency which allows administration of the video capsule as first mentioned.

If a narrow area exists, the condition and the position of the bottleneck in especially the small intestine can be assessed and identified prior to operation, with a double balloon endoscope as described in Germany Patent Application Publication No. 10 2004 023 457.

Materials with predetermined dissolution times are sufficiently well-known in the pharmaceutical industry. Thus, for example, a capsule for treating "Crohn's disease" dissolves precisely in the "ileocecal region" after passing the stomach, the duodenum and the small intestine. And then, the capsule discharges the medicine "mesalazin" for treatment.

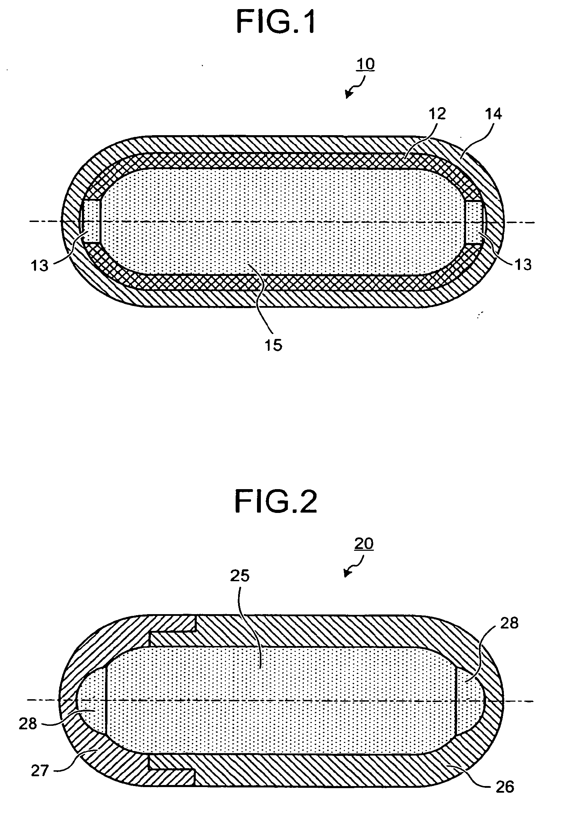

An example of the first embodiment will be described below with reference to Figs. 1 to 3. Fig. 1 shows a medical capsule 10 for the penetrability testing of the intestinal tract. The medical capsule 10 includes an inner casing 12 which has two discharge outlets 13 which face each other, and a sealed outer casing 14. The inner casing 12 and the outer casing 14 are made of a material which is dissolved by the digestive secretions of the intestinal tract. The inner casing 12 is filled with a filling 15 of a granulated material, powder, gel or liquid. The outer casing 14 surrounds an outer surface of the inner casing 12 and has a substantially uniform thickness. The inner casing 12 has an inner space similar to the outline of the inner casing 12 and/or the outer casing 14.

Fig. 2 shows a medical capsule 20 for testing patency of the intestinal tract. The medical capsule 20 is formed from a filled casing made of a material which is dissolved by the digestive secretions of the intestinal tract. This casing includes a lower portion 26 and an upper portion 27 which are hermetically joined to each other. The wall thickness of the casing at both ends is designed to thin by shaping the interior (shaped portion 28). The filled casing is filled with the same material as the filling 15.

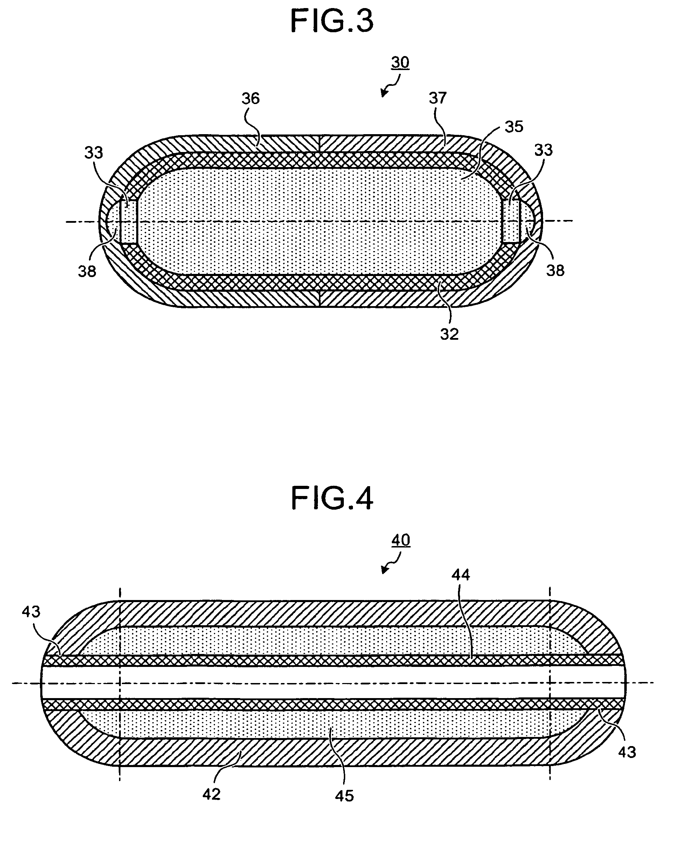

Fig. 3 shows a medical capsule 30 for testing patency of the intestinal tract. The medical capsule 30 includes an inner casing 32 with two discharge outlets (openings) facing each other and an outer casing formed by two cup-shaped members 36 and 37. The inner and outer casings 32 are made of a material which is dissolved by digestive secretions of the intestinal tract. The inner casing 32 is filled with a filling 35 made of the same material as the filling 15. The wall thickness of the outer casing at both ends may be designed to thin by shaping the interior (shaped portion 38) as shown in Fig. 1.

The medical capsule according to the first embodiment can assess whether a narrow area exists in the intestinal tract by a reliable method. This is achieved by the capsule, which is formed from the inner casing and the outer casing which are made of a material which is completely dissolved after a predetermined time by the digestive secretions of the intestinal tract. The inner casing is filled with a filling. The filling can flow into the intestinal tract through two facing discharge outlets after the outer casing dissolves.

However, there is a problem that the medical capsule can completely plug the narrow-area when reaching a narrow area in the intestinal tract. This may result in flatulence, so that the capsule cannot be excreted naturally. Or there may be an accumulation of liquid as a filling in a narrow area, so that the capsule cannot be excreted. These cases may result in emergency surgery.

A medical capsule according to a second embodiment is for testing patency of an intestinal tract, and it includes a discharge path or through-bore which is useful for ensuring flow path to certainly find and specify a narrow area in the intestinal tract.

A basic concept of the second embodiment is to provide a medical capsule which includes a hollow body made of a material which is dissolved after a predetermined time by the digestive secretions of the intestinal tract, and the medical capsule includes bores at both ends and a contrast medium.

A continuous tube made of the same material as the hollow body is inserted into the bores. The continuous tube includes no contrast medium and closes the hollow body. The continuous tube also includes a thinner wall than the hollow body and, therefore, dissolves earlier than the hollow body at a narrow area of the intestinal tract.

A space filled with a filling exists between an outer diameter of the continuous tube and an inner diameter of the hollow body. After dissolution of the tube by the intestinal secretions, the filling flows into the intestinal tract, resulting in a signal to the patient or an external device. However, the hollow body remains at the narrow area for a predetermined time required to completely dissolve the hollow body. The position of the narrow area can be detected by X-ray technology with a contrast medium contained in the hollow body.

A low-cost example of the medical capsule according to the second embodiment is one formed by a hollow body having only two facing bores without a tube or a filling. The hollow body dissolves after a predetermined time by the secretions of the intestinal tract, and contains a contrast medium. This medical capsule does not generate the above-mentioned signal at the narrow area. The presence of a narrow area in the intestinal tract is determined whether a medical capsule is contained in the stool extracted after an average time. Visibility of the medical capsule in the stool is improved if the medical capsule has a unique colour. If the medical capsule is not excreted after a pre-given time, its position in the intestinal tract can be determined because of the contrast medium by X-ray.

Another example of the medical capsule according to the second embodiment is one formed by a body having a penetrating slender bore (through-bore). This body dissolves by intestinal secretions after a pre-given time. Also a contrast medium may be added in the material.

The example of the second embodiment is explained in detail below with reference to Figs. 4 to 6. Fig. 4 shows a medical capsule 40 for testing patency of the intestinal tract. The medical capsule 40 includes a hollow body 42 made of a material which is dissolved by secretions of the intestinal tract. The hollow body 42 has bores 43 at both ends and a through-bore formed with the bore 43. The hollow body 42 contains a contrast medium as a material. A continuous tube 44 is made of a material which is dissolved by the secretions of the intestinal tract, and is inserted into the bores 43. The continuous tube 44 has a thin wall which seals a hollow portion of the hollow body 42. A space 45 is formed between the continuous tube 44 and the hollow body 42, and is filled with a filling. The filling flows into the intestinal tract after the continuous tube 44 dissolves.

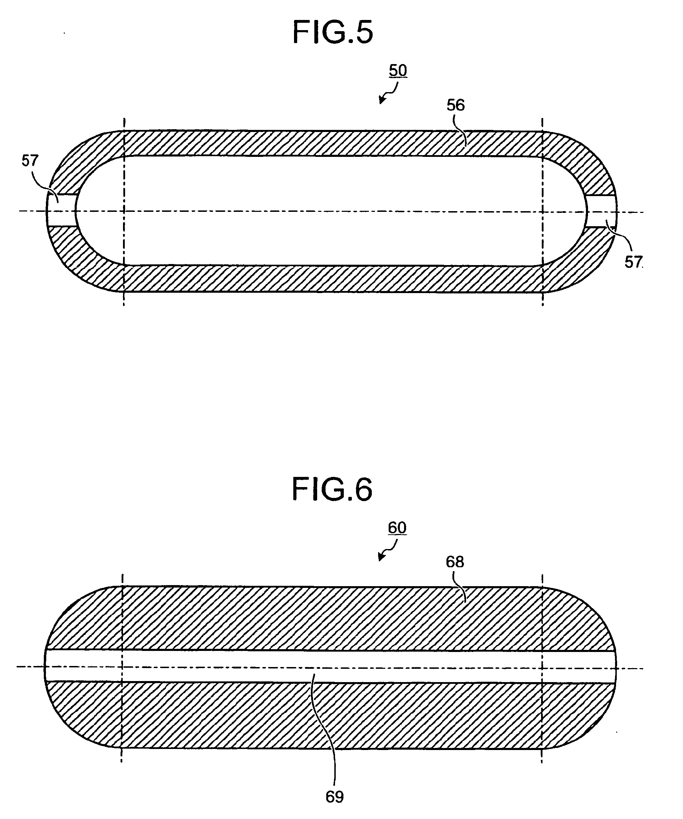

Fig. 5 shows a medical capsule 50 including a hollow body 56 having two facing bores 57. The hollow body 56 includes a material which is dissolved by the secretions of the intestinal tract, and contains a contrast medium.

Fig. 6 shows a medical capsule 60 includes a body 68 with a slender bore 69. The body 68 is made of a material which is dissolved by the secretions of the intestinal tract, and contains a contrast medium.

A medical capsule according to a third embodiment is explained below. A basic concept of the third embodiment is to provide a medical capsule which includes a hollow body made of a material which is dissolved after a predetermined time by the digestive secretions of the intestinal tract. The hollow body has bores at both ends and contains a contrast medium. The bores are closed with plugs (promptly-dissolving portion) which are resistant to the secretion of the stomach. The plugs are dissolved, for example, in six to eight hours after the medical capsule pass the stomach. The medical capsule is filled with liquid dye or powder dye. The liquid dye or the powder dye flows out of the medical capsule into the medium gastrointestine after the plugs dissolved, and colours an inner wall or the mucosa of the small intestine. The plug can be also one plug having such a shape as to close over the entire length of the through bore formed by the bores or having a pipe-shape.

The hollow body is designed to completely dissolve by the secretions of the intestine within 30 to 40 hours. If the hollow body is found in the stool, it will be determined that a gastrointestinal tract has enough patency to administer a video capsule. On the other hand, if the capsule does not show up in the stool after 20 to 25 hours, whether the capsule stuck at a narrow-area can be examined by X-ray.

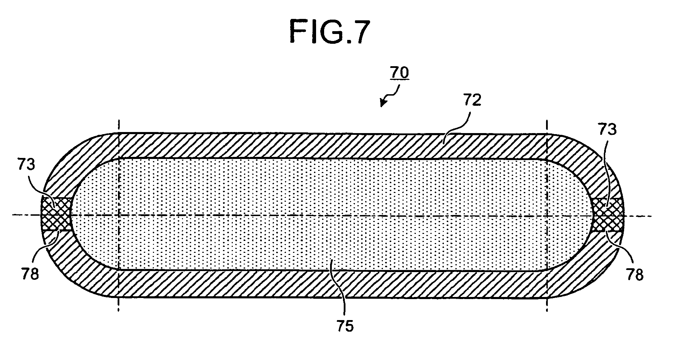

An example of the third embodiment is explained below with reference to Fig. 7. Fig. 7 shows a medical capsule 70 for colouring the medium gastrointestinal tract. The medical capsule 70 includes a hollow body 72 made of a material which is dissolved by the secretions of an intestinal tract. The hollow body 72 has bores 78 at both ends. The hollow body 72 also contains a contrast medium. Each bore 78 is closed with a plug 73. The plug 73 is made of a material which is resistant to the secretions of the stomach; however, it dissolves by the secretions of the intestine tract within approximately six to eight hours. The medical capsule 70 is filled with the above-mentioned filling such as a liquid dye 75, a powder dye, or the like. The filling flows into the middle gastrointestinal tract after the plug 73 dissolves.

### INDUSTRIAL APPLICABILITY

As described above, the medical capsule according to the present invention is useful for testing patency of the intestine tract and treatments of the gastrointestinal before a VIDEO capsule is administered. The medical capsule is also used for a medical system including the VIDEO capsule.

## Claims

1. A medical capsule (10; 30) having a capsule shape and size capable of being introduced into a subject body, the medical capsule (10; 30) comprising a casing having the capsule shape, made of a material, which is dissolvable by remaining in a lumen of the subject body for at least a given period of time, and having at least two thin wall portions, thickness of each of the thin wall portions being thinner than that of the rest of the casing,
wherein the casing is formed by an inner casing (12, 32) with a plurality of discharge outlets (13, 33) and an outer casing (14, 36, 37) with a substantially uniform thickness, the outer casing (14, 36, 37) surrounding an outer surface of the inner casing (12, 32);
the thin wall portions are portions of the outer casing (14, 36, 37) which face the discharge outlets (13, 33) of the inner casing (12, 32),
a communication opening for communicatively connecting an inner space of the casing to an outside of the medical capsule (10; 30) is formed when the thin wall portions are eliminated earlier than other portions of the medical capsule (10; 30) by dissolution of the casing, and
the casing contains a contrast medium visible by X-ray as a material.

2. The medical capsule (30) according to claim 1, wherein
the thin wall portions are formed so that an interior of a wall of the outer casing (36, 37) is shaped at positions facing the discharge outlets (33) of the inner casing (32).

3. The medical capsule (30) according to claim 1 or 2, wherein the casing is formed by two cup-shaped members (36, 37) which are connected to each other.

4. The medical capsule (10; 30) according to claim 1, wherein the contrast medium is homogeneously-mixed and added to the material of the casing.

5. The medical capsule (10; 30) according to claim 1, wherein the contrast medium is barium sulphate.

6. The medical capsule (10; 30) according to any one of claims 1 to 5, wherein the space is substantially similar to an outline of the casing.

7. The medical capsule (10; 30) according to any one of claims 1 to 6, wherein the space is filled with a filling of at least one of granulated material, powder, gel and liquid.

8. The medical capsule (10; 30) according to any one of claims 1 to 6, wherein the space is filled with a filling of at least one of gas with an odor, gel, liquid dye, painkiller, and other medicine.

## Patentansprüche

1. Medizinische Kapsel (10; 30) mit einer Kapselform und einer zum Einführen in einen Körper eines Objekts geeigneten Größe, wobei die medizinische Kapsel (10; 30) ein Gehäuse mit einer Kapselform aufweist, wobei das Gehäuse aus einem Material hergestellt ist, das durch Verbleiben im Lumen eines Objektkörpers für wenigstens eine vorbestimmte Zeitspanne auflösbar ist und wenigstens zwei dünne Wandabschnitte aufweist, wobei die Dicke jedes der dünnen Wandabschnitte dünner als der Rest des Gehäuses ist,
wobei das Gehäuse von einem inneren Gehäuse (12, 32) mit einer Vielzahl von Auslassdurchgängen (13, 33) und einem äußeren Gehäuse (14, 36, 37) mit einer im Wesentlichen gleichförmigen Dicke gebildet ist, wobei das äußere Gehäuse (14, 36, 37) eine Außenfläche des inneren Gehäuses (12, 32) umgibt;
die dünnen Wandabschnitte Abschnitte des äußeren Gehäuses (14, 36, 37) sind, die den Auslassdurchgängen (13, 33) des inneren Gehäuses (12, 32) gegenüberliegen,
eine Kommunikationsöffnung zur kommunikativen Verbindung eines Innenraums des Gehäuses mit der Außenseite der medizinischen Kapsel (10; 30) wird gebildet, wenn die dünnen Wandabschnitte vor den anderen Abschnitten der medizinischen Kapsel (10; 30) durch Auflösen des Gehäuses entfernt werden, und
das Gehäuse ein durch Röntgenstrahlung sichtbares Kontrastmedium als ein Material enthält.

2. Medizinische Kapsel (30) nach Anspruch 1, wobei
die dünnen Wandabschnitte derart gebildet sind, dass das Innere einer Wand des äußeren Gehäuses (36, 37) an Positionen geformt wird, die den Auslassdurchgängen (33) des inneren Gehäuses (32) gegenüber liegen.

3. Medizinische Kapsel (30) nach einem der Ansprüche 1 oder 2, wobei das Gehäuse durch zwei kalottenförmige Elemente (36, 37) gebildet wird, die miteinander verbunden sind.

4. Medizinische Kapsel (10; 30) nach Anspruch 1, wobei das Kontrastmedium homogen gemischt ist und dem Material des Gehäuses zugefügt wird.

5. Medizinische Kapsel (10; 30) nach Anspruch 1, wobei das Kontrastmedium Bariumsulfat ist.

6. Medizinische Kapsel (10; 30) nach einem der Ansprüche 1 bis 5, wobei der Raum im Wesentlichen gleich zur Gestalt des Gehäuses ist.

7. Medizinische Kapsel (10; 30) nach einem der Ansprüche 1 bis 6, wobei der Raum mit einer Füllung von wenigstens einem granularen Material, einem Puder, einem Gel und einer Flüssigkeit gefüllt ist.

8. Medizinische Kapsel (10; 30) nach einem der Ansprüche 1 bis 6, wobei der Raum mit einer Füllung von wenigstens einem Gas mit einem Duft, einem Gel einer flüssigen Färbung, einem Schmerzmittel oder einer anderen Medizin gefüllt ist.

## Revendications

1. Capsule médicale (10; 30) ayant une forme de capsule et une taille apte à être introduite dans un corps de sujet, la capsule médicale (10; 30) comprenant un boîtier ayant la forme de capsule, constitué d'un matériau, qui est soluble en restant dans une lumière du corps de sujet pendant au moins une période de temps donnée, et ayant au moins deux parties de paroi mince, une épaisseur de chacune des parties de paroi mince étant plus mince que celle du reste du boîtier,
dans laquelle le boîtier est formé par un boîtier intérieur (12, 32) avec une pluralité de sorties de décharge (13, 33) et un boîtier extérieur (14, 36, 37) avec une épaisseur sensiblement uniforme, le boîtier extérieur (14, 36, 37) entourant une surface extérieure du boîtier intérieur (12, 32);
les parties de paroi mince sont des parties du boîtier extérieur (14, 36, 37) qui font face aux sorties de décharge (13, 33) du boîtier intérieur (12, 32),
une ouverture de communication pour connecter en communication un espace intérieur du boîtier avec un extérieur de la capsule médicale (10; 30) est formée lorsque les parties de paroi mince sont éliminées plus tôt que d'autres parties de la capsule médicale (10; 30) par dissolution du boîtier, et
le boîtier contient un milieu de contraste visible par rayons X comme un matériau.

2. Capsule médicale (30) selon la revendication 1, dans laquelle
les parties de paroi mince sont formées de telle manière qu'un intérieur d'une paroi du boîtier extérieur (36, 37) est formé en des positions faisant face aux sorties de décharge (33) du boîtier intérieur (32).

3. Capsule médicale (30) selon la revendication 1 ou 2, dans laquelle le boîtier est formé par deux éléments (36, 37) en forme de coupelle qui sont connectés l'un à l'autre.

4. Capsule médicale (10; 30) selon la revendication 1, dans laquelle le milieu de contraste est mélangé de façon homogène et ajouté au matériau du boîtier.

5. Capsule médicale (10; 30) selon la revendication 1, dans laquelle le milieu de contraste est du sulfate de baryum.

6. Capsule médicale (10; 30) selon l'une quelconque des revendications 1 à 5, dans laquelle l'espace est sensiblement similaire à un profil du boîtier.

7. Capsule médicale (10; 30) selon l'une quelconque des revendications 1 à 6, dans laquelle l'espace est rempli avec une garniture d'au moins un parmi un matériau granulé, une poudre, un gel et un liquide.

8. Capsule médicale (10; 30) selon l'une quelconque des revendications 1 à 6, dans laquelle l'espace est rempli avec une garniture d'au moins un gaz avec une odeur, un gel, un colorant liquide, un antalgique, ou autre médicament.
